# EUROPÄISCHE PATENTSCHRIFT

(11) **EP 1 905 412 B1**
(45) Veröffentlichungstag und Bekanntmachung des Hinweises auf die Patenterteilung: **11.05.2011**
(21) Anmeldenummer: 06020553.1
(22) Anmeldetag: 29.09.2006
(51) Int. Cl.: A61K 6/027, C03C 3/091

(54) **Glas für dentale Anwendungen**
Glass for dental uses
Verre pour utilisation dentaire

(43) Veröffentlichungstag der Anmeldung: 02.04.2008
(73) Patentinhaber: Ivoclar Vivadent AG, 9494 Schaan (LI)
(72) Erfinder: Tauch, Diana Dr., 8887 Mels (CH); Bürke, Harald Dr., 6820 Frastanz (AT); Rheinberger, Volker M. Dr., 9490 Vaduz (LI)
(74) Vertreter: UEXKÜLL & STOLBERG

(56) Entgegenhaltungen:
- EP-A- 1 170 261
- EP-A- 1 452 500
- EP-A1- 0 544 145
- EP-A1- 0 622 342
- EP-A1- 0 695 726
- EP-A1- 0 827 941
- EP-A1- 1 167 311
- EP-A2- 0 885 606
- EP-A2- 1 329 430
- WO-A2-99/45888
- DE-A1- 10 336 913

## Beschreibung

Die Erfindung betrifft ein Glas für dentale Anwendungen, das einen niedrigen Wärmeausdehnungskoeffizienten und eine hohe Brennstabilität aufweist und sich ausgezeichnet als Glasurmaterial für dentale Restaurationen eignet.

In der Zahnheilkunde werden neben metallgestützten Restaurationen zunehmend vollkeramische Restaurationen verwendet. Die Gerüste aus Metall oder Keramik werden zur Erzielung ästhetischer besonders anprechender Restaurationen üblicherweise mit mehreren Schichten aus Beschichtungs- und Verblendmaterialien versehen.

Es hat sich bei der Verarbeitung von keramischen Gerüsten nun ein Verfahren etabliert, bei dem sowohl nach Durchführung der Schichttechnik als auch der Maltechnik ein separater Glasurbrand bei hohen Temperaturen erfolgt. Die dadurch erhaltene Glasur hat neben der Erzielung eines optisch ansprechenden Glanzes auch die Aufgabe, mögliche Unebenheiten der Oberfläche auszugleichen und somit die Plaquebildung zu vermindern. Das Aufbringen dieser Glasur stellt aber nicht nur einen weiteren Arbeitsschritt, sondern auch eine weitere thermische Belastung der bis dahin bearbeiteten dentalen Restauration dar.

Aus dem Stand der Technik sind die verschiedensten Glaskeramiken bekannt, die sich als Gerüstmaterial für dentale Restaurationen eignen. Für die Herstellung dieser Gerüste ist in den vergangenen Jahren neben der Heisspresstechnik (z.B. IPS Empress^{®} der Ivoclar Vivadent AG, Schaan, Liechtenstein) auch die mechanische Bearbeitung in CAD/CAM-Anlagen hinzugekommen. Dabei hat insbesondere das CEREC^{®}-System der Firma Sirona weltweit viele Anwender gefunden.

Weiterhin sind aus dem Stand der Technik Glaskeramiken bekannt, die zur einfachen mechanischen Bearbeitung in einer ersten Kristallisationsstufe vorliegen und nach dieser Bearbeitung einer weiteren Kristallisation, auch als Keramisierung bezeichnet, unterzogen werden, um die mechanischen Eigenschaften des Gerüstes der dentalen Restauration zu verbessern. Beispiele für solche Gerüstmaterialien sind Glimmer-Glaskeramiken oder Lithiumsilikat-Glaskeramiken, die im Zustand der Metasilikatphase mechanisch bearbeitet werden und anschliessend durch eine weitere Keramisierung die Lithiumdisilikatphase ausbilden und dann eine deutlich höhere Festigkeit aufweisen. Solche Glaskeramiken sind in der DE 103 36 913 ausführlich beschrieben.

Als weitere Materialien haben in den letzten Jahren verschiedene Qualitäten von stabilisierten ZrO₂-Keramiken Eingang in die zahntechnischen Labors und auch in die Zahnarztpraxen gefunden. Hierbei haben sich, jeweils in Abhängigkeit des Sintergrades, d.h. teil- oder vollständig dichtgesinterte Materialien unterschiedliche Maschinen- und Bearbeitungssysteme auf dem Markt etabliert.

Zur Erzielung einer dem natürlichen Zahn entsprechenden Ästhetik werden nun solche keramischen oder auch metallischen Gerüste mit mehreren Schichten aus Glaskeramiken oder Gläsern verblendet und abschliessend mit einer Glasur versiegelt.

Die EP 1170261 offenbart eine Kalium-Zink-Silikat Glaszusammensetzung, die sich als Beschichtungs- und Verblendmaterial für keramische Dentalsuprastrukturen eignet.

Die EP 1329430 offenbart Alkali-Zink-Silicat-Glaskeramiken und -Gläser, die sich allgemein als Dentalmaterial und insbesondere zur Verblendung von Dentalrestaurationen eignen.

Es gibt jedoch eine Reihe von Ausgangsmaterialien, bei denen es nicht erforderlich ist, durch zusätzliches Aufbringen mehrere Schichten das Aussehen des natürlichen Zahns zu simulieren. Hierbei sind vor allem mehrfarbige Rohlinge für die CAD/CAM-Verarbeitung zu nennen, wie sie von der Firma Vita unter der Bezeichnung Trilux^{®} vertrieben. Es ist davon auszugehen, dass in Kürze das Interesse an solchen Ausgangsmaterialien stark ansteigen wird, da sich dadurch eine immense Zeitersparnis bei der Herstellung von Dentalrestaurationen ergibt.

Ebenso ist es möglich, geeignet eingefärbte Ausgangsmaterialien zu verarbeiten, wenn die daraus hergestellte Restauration nicht im direkt sichtbaren Bereich liegt. Hierbei sind besonders Inlays oder Onlays im Seitenzahnbereich zu nennen. Diese Restaurationen lassen es zu, dass nur mit einer Glasur eine zahntechnische Fertigstellung möglich ist.

Nun hat es sich aber auch gezeigt, dass immer mehr Materialien eingesetzt werden, die für eine leichtere Bearbeitbarkeit mittels CAD/CAM in einem Zustand verarbeitet werden, in dem noch tels CAD/CAM in einem Zustand verarbeitet werden, in dem noch nicht die höchste Festigkeit erreicht ist. Nach dem Fräsen bzw. Schleifen wird eine weitere Kristallisation durch thermische Behandlung ausgelöst und somit werden die mechanischen Eigenschaften verbessert. Sofern ebenfalls die üblichen Verblend- und Schichtmaterialien verwendet werden, ist für die gewünschte Glasur noch ein gesonderter Brand notwendig, da die bekannten Verblend- und Schichtmaterialien nicht über die erforderliche Brennstabilität verfügen. Diesen üblichen Materialien mangelt es ebenfalls an der erforderlichen Formstabilität, bei den zum Aufbrennen benutzten hohen Temperaturen. Das äussert sich darin, dass diese Materialien zu fließen beginnen.

Der Erfindung liegt somit die Aufgabe zugrunde, ein Material bereitzustellen, mit dem eine Glasur auf einer dentalen Restauration erzeugt werden kann und welches eine hohe Formstabilität beim Aufbrennen hat. Darüber hinaus soll die Verarbeitung dieses Material zu einer Glasur gleichzeitig die Bildung von Kristallen in einer dazu geeigneten Restauration bewirken und somit gestatten, dass die Arbeitsschritte der (a) abschliessende Keramisierung bzw. Kristallisation und des (b) Glasurbrand in einem Arbeitsschritt durchgeführt werden können.

Diese Aufgabe wird überraschenderweise durch das Glas für dentale Anwendungen gemäß den Ansprüchen 1 bis 9 gelöst.

Die Erfindung betrifft ebenfalls die Verwendung des Glases nach den Ansprüchen 10 bis 14 sowie das Verfahren zum Aufbringen einer Glasur auf eine dentale Restauration nach den Ansprüchen 15 bis 20.

Das erfindungsgemäße Glas für dentale Anwendungen zeichnet sich dadurch aus, dass es die folgenden Komponenten enthält

| Komponente | Gew.-% |
|---|---|
| SiO₂ | 60,0 - 65,0 |
| K₂O | 15,0 - 19,0 |
| Al₂O₃ | 6,0 - 10,5 |
| Li₂O | 1,8 - 2,2 |
| Me^{II}O | 2,5 - 8,5 |
| Weitere Oxide | 1,5 - 7,0 |

wobei
(a) Me^{II}O für mindestens ein zweiwertiges Oxid steht, das ausgewählt ist aus der Gruppe der Oxide von Ca, Mg, Sr und Zn, und
(b) weitere Oxide für mindestens ein Oxid steht, das aus der Gruppe der Oxide von B, Zr, Ce und Y ausgewählt ist.

Dabei ist es bevorzugt, dass das Glas im wesentlichen aus den Komponenten besteht.

Weiter ist es bevorzugt, wenn das Glas mindestens eine der Komponenten in der folgenden Menge enthält

| Komponente | Gew.-% |
|---|---|
| SiO₂ | 63,0 - 65,0 |
| K₂O | 17,5 - 19,0 |
| Al₂O₃ | 9,5 - 10,5 |
| Li₂O | 1,9 - 2,1 |
| Me^{II}O | 4,5 - 7,5 |
| Weitere Oxide | 2,5 - 6,0. |

Es hat sich ebenfalls als günstig erwiesen, wenn das Glas weniger als 2,0 Gew.-% an ZnO enthält.

Weiter ist ein Glas bevorzugt, das 0,5 bis 2,5 Gew.-% an ZrO₂ enthält.

In einer weiteren bevorzugten Ausführungsform enthält das Glas 15,5 bis 19,0, insbesondere 17,5 bis 19,0 Gew.-% an K₂O.

Besonders bevorzugt ist ein Glas, das mindestens eine der Komponenten in der folgenden Menge enthält

| Komponente | Gew.-% |
|---|---|
| SiO₂ | 64.0 - 65.0 |
| K₂O | 18.0 - 18.5 |
| Al₂O₃ | 10.0 - 10.5 |
| Li₂O | 2.0 - 2.1 |
| MgO | 0.4 - 2.1, insbesondere 0.4 - 0.7 |
| CaO | 1.9 - 2.3, insbesondere 1.9 - 2.1 |
| ZrO₂ | 1.0 - 2.4, insbesondere 2.0 - 2.4 |
| CeO₂ | 0.4 - 0.8. |

Das erfindungsgemäße Glas erweist sich überraschenderweise als formstabil bei den hohen Temperaturen, die zu einer gleichzeitigen Kristallbildung einer geeigneten dentalen Restauration verwendet werden. Somit kann die abschließende Keramisierung eines geeigneten Gerüstmaterials gleichzeitig mit der gewünschten Aufbringung einer Glasur in einem Schritt erfolgen. Dies stellt einen enormen Vorteil für den Anwender dar.

Von besonderen Vorteil ist die Verwendung des erfindungsgemässen Glases auf Restaurationsteilen, die in einem nur teilweise verfestigtem Zustand geschliffen oder gefräst wurden und nachträglich einer weiteren thermischen Behandlung unterzogen werden, um die mechanischen Eigenschaften zu verbessern. Insbesondere ist die Verwendung des Glases auf Restaurationsteilen aus einer Lithiummetasilikat-Glaskeramik als Glasur geeignet, die infolge der thermischen Behandlung Lithiumdisilicat-Kristalle bildet.

Weiter hat das erfindungsgemäße Glas bevorzugt eine Brenntemperatur von 900 bis 950°C, insbesondere von 910 bis 930°C. Die Brenntemperatur wird in der in den Beispielen angegebenen Weise bestimmt.

Darüber hinaus geht das erfindungsgemäße Glas keine Reaktionen mit dem Gerüstmaterial ein und verleiht ihm nach der thermischen Behandlung einen ästhetischen hohen Glanz. Es besitzt außerdem eine sehr gute chemische Stabilität. Dies ist eine wichtige Eigenschaft für Materialien die in der Mundhöhle permanent in Kontakt mit unterschiedlichsten Fluiden kommen.

Bei Verwendung als Glasurmaterial für ein Restauration auf Basis eines Glases oder einer Glaskeramik kann der Wärmeausdehnungskoeffizient des erfindungsgemäßen Glases durch Variation der Art und Mengen der im Glas vorhandenen Komponenten geeignet angepaßt werden. Bevorzugt ist ein Glas, das einen linearen thermischen Ausdehnungskoeffizienten von 8,0 bis 9,5 · 10⁻⁶ K⁻¹ und insbesondere von 8,4 bis 9,1 · 10⁻⁶ K⁻¹ im Temperaturbereich von 100 bis 500°C hat.

Das erfindungsgemäße Glas kann nach den aus dem Stand der Technik bekannten und üblichen Verfahren hergestellt werden, indem aus geeigneten Ausgangsmaterialien, wie Oxiden, Hydroxiden und Carbonaten, eine Glasschmelze bei Temperaturen von etwa 1200°C bis etwa 1600°C gebildet wird.

Üblicherweise wird die Glasschmelze dann in Wasser gegossen und das dabei gebildete Glasgranulat wird auf eine Korngrösse von maximal 100 µm, vorzugsweise maximal 50 µm zerkleinert.

Das erfindungsgemäße Glas wird insbesondere als Glasurmaterial von einer dentalen Restauration verwendet und die Erfindung ist ebenfalls auf diese Verwendung gerichtet.

Dabei handelt es sich bei der dentalen Restauration insbesondere um eine Brücke, eine Krone, ein Inlay, ein Onlay, eine Verblendung oder eine Kappe, oder ein Teil von diesen.

Die dentale Restauration ist vorzugsweise auf der Basis eines Glases, einer Keramik, eines Metalls oder einer Glaskeramik und insbesondere auf Basis einer Glaskeramik.

Als Glaskeramik wird dabei vorzugsweise Lithiumsilicat-Glaskeramik, eine Glimmer-Glaskeramik oder eine Chondrodit-Glaskeramik eingesetzt. Diese können aus geeigneten Vorläufern hergestellt werden, die nach dem Aufbringen des erfindungsgemäßen Glases als Glasur infolge der thermischen Behandlung und der dadurch ausgelösten Kristallbildung eine starke Zunahme der Festigkeit zeigen und zur Bildung der gewünschten Glaskeramik führen.

Die Lithiumsilicat-Glaskeramik ist vorzugsweise eine Lithiummetasilicat- oder eine Lithiumdisilicat-Glaskeramik.

Bei Einsatz einer Lithiummetasilicat-Glaskeramik als Vorläufer führt die thermische Behandlung zur Umwandlung der Metasilicat-Kristalle zu Disilicat-Kristallen, wodurch das Gefüge stark verfestigt wird.

Die Erfindung ist dementsprechend auch auf ein Verfahren zum Aufbringen einer Glasur auf eine dentale Restauration gerichtet, bei dem
(a) das erfindungsgemäße Glas auf die dentale Restauration aufgebracht und
(b) die mit dem Glas versehende dentale Restauration einer thermischen Behandlung unterzogen wird, um das Glas in eine auf der Restauration fest haftende Glasur umzuwandeln.

Dabei erfolgt die thermische Behandlung vorzugsweise bei einer Temperatur von wenigstens 860°C, insbesondere bei 860 bis 960°C.

Wie bereits erwähnt wird dabei insbesondere eine dentale Restauration auf der Basis eines Glases, einer Keramik, eines Metalls oder einer Glaskeramik verwendet.

Es ist besonders bevorzugt, wenn die thermische Behandlung auch zur Bildung von Kristallen in dem Glas oder der Glaskeramik führt. Unter Bildung von Kristallen wird auch die Umwandlung von Kristallen, z.B. Umwandlung von Metasilicat- zu Disilicat-Kristallen, verstanden.

Das erfindungsgemäße Glas bietet nicht nur bei Verwendung in Verfahren zur Aufbringung einer Glasur auf Lithiumsilikat-Glaskeramiken große Vorteile, sondern es kann in vorteilhafter Weise auch bei anderen glaskeramischen Materialien eingesetzt werde, die je nach thermischer Behandlung unterschiedliche Kristallphasen ausscheiden. Beispiele solcher anderer glaskeramischer Materialien sind angegeben in K. Chyung, G.H. Beall, D.G. Grossman; Fluorophlogopite Mica Glass-Ceramics; X. Int. Cong. on Glass, Kyoto, The Ceramic Soc. Japan 14 (1974) 33-40.

In dem erfindungsgemäßen Verfahren werden bevorzugt solche Gläser oder Glaskeramiken verwendet, die bei der thermischen Behandlung Lithiumdisilicat-Kristalle oder Glimmer-Kristalle bilden können. Deshalb wird in Schritt (a) vorzugsweise eine Restauration auf Basis von Lithiummetasilicat- oder Chondrodit-Glaskeramik eingesetzt.

Weiter ist ein Verfahren besonders bevorzugt, bei dem die Bildung von Kristallen eine Steigerung der Festigkeit, insbesondere der Biegefestigkeit, der dentalen Restauration bewirkt.

Die Erfindung wird im folgenden anhand von Beispielen näher erläutert:

### Beispiele

### Beispiel 1 bis 4 Zusammensetzung und Eigenschaften erfindungsgemäßer Gläser

Es wurden insgesamt 4 verschiedene erfindungsgemäße Gläser mit den in der nachstehenden Tabelle angegebenen chemischen Zusammensetzungen hergestellt:

**Tabelle 1: Zusammensetzung erfindungsgemäßer Gläser (Angaben in Gew.-%)**

| Komponente | Beispiel 1 | Beispiel 2 | Beispiel 3 | Beispiel 4 |
|---|---|---|---|---|
| SiO₂ | 60,69 | 64,46 | 64,86 | 61,57 |
| K₂O | 16,63 | 18,20 | 18,31 | 15,78 |
| Al₂O₃ | 6,53 | 10,07 | 8,05 | 6,20 |
| Li₂O | 2,11 | 2,01 | 2,03 | 2,00 |
| MgO | 0,53 | 0,54 | 2,01 | 0,50 |
| CaO | 2,27 | 2,01 | 2,03 | 2,15 |
| SrO | 2,11 | - | - | 2,00 |
| ZnO | 3,16 | - | - | 3,00 |
| B₂O₃ | - | - | - | 1,13 |
| ZrO₂ | 1,05 | 2,16 | 2,17 | 1,00 |
| CeO₂ | 0,71 | 0,54 | 0,54 | 0,67 |
| Y₂O₃ | 4,22 | - | - | 4,00 |

Zu ihrer Herstellung wurde jeweils ein Gemenge von geeigneten Oxiden, Hydroxiden und Carbonaten in einem Platin/Rhodium-Tiegel bei einer Temperatur von 1500°C bis 1550°C während einer Homogenisierungszeit von 1 bis 1,5 Stunden erschmolzen. Die Glasschmelze wurde in Wasser abgeschreckt und auf eine mittlere Korngrösse von kleiner als 100 µm, bezogen auf die Anzahl der Teilchen, aufgemahlen.

Für die Gläser aus der Tabelle 1 sind in Tabelle 2 ausgewählte Eigenschaften angegeben, die an geeigneten Prüfkörpern aus dem jeweiligen Glas bestimmt worden sind. Die Beispiele verdeutlichen, dass durch Variation der Komponenten und ihrer Mengen in den angegebenen Bereichen Gläser mit den gewünschten Eigenschaften hergestellt werden können.

**Tabelle 2: Eigenschaften erfindungsgemäßer Gläser**

| Beispiel Nr. | Brenntemperatur | Ausdehnungskoeffizient (100-500 °C) | Chemische Beständigkeit | Vickers-Härte HV5 |
|---|---|---|---|---|
| 1 | 910°C | 8,95 · 10⁻⁶ K⁻¹ | 1 µg/cm² | 5463 MPa |
| 2 | 930°C | 8,87 · 10⁻⁶ K⁻¹ | 6 µg/cm² | 5804 MPa |
| 3 | 930°C | 9,07 · 10⁻⁶ K⁻¹ | 3 µg/cm² | 5382 MPa |
| 4 | 920°C | 8,44 · 10⁻⁶ K⁻¹ | 8 µg/cm² | 5310 MPa |

Die Bestimmung dieser Eigenschaften erfolgte in der nachfolgend beschriebenen Art und Weise unter Verwendung der angebenen Normen:

### Bestimmung der Brenntemperatur

Die Brenntemperatur wurde an Plättchen mit einem Durchmesser von 12 mm und einer Höhe von 2 mm bestimmt, welche nach DIN ISO 6872 hergestellt wurden. Diese Plättchen wurden bei unterschiedlichen Temperaturen gebrannt und anschliessend wurde der Brenngrad an Hand der Kriterien Blasigkeit, Oberflächenglanz und Kantenabrundung beurteilt.

### Bestimmung des Ausdehnungskoeffizienten α

Zur Messung des linearen thermischen Ausdehnungskoeffizienten α nach ISO 6872 wurde aus dem Pulver des jeweiligen Glases ein stäbchenförmiger Grünkörper hergestellt, der in einem Vakuumbrennofen mit einer Aufheizrate von 60 °C/min und einer Haltezeit von 1 min bei der in Tabelle 2 angegebenen Brenntemperatur für die Prüfkörperherstellung gesintert wurde. An den so erhaltenen Probekörpern wurde anschliessend der lineare thermische Ausdehnungskoeffizient im Temperaturbereich von 100 °C bis 500 °C bestimmt.

### Bestimmung der chemischen Beständigkeit

Die Säurebeständigkeit ist ein Mass für die chemische Beständigkeit gerade von im Dentalbereich eingesetzten Gläsern, da diese in der Mundhöhle permanent der Einwirkung von sauren Substanzen ausgesetzt sind.

Die Säurebeständigkeit wurde gemäss ISO 6872 (1995) bestimmt. Dazu wurden zunächst Probeplättchen mit einem Durchmesser von 12 mm und einer Dicke von 1 mm durch Zusammensintern des Glaspulvers mit einer mittleren Teilchengrösse von weniger als 50 µm hergestellt. Das Pulver wurde 1 Minute lang auf der jeweiligen Brenntemperatur für die Prüfkörperherstellung gehalten. Dann wurden die Probeplättchen 16 Stunden lang mit 4 Vol.-%iger wässriger Essigsäure bei 80 °C behandelt. Es wurde anschliessend der eingetretene Masseverlust bezogen auf die Oberfläche der Prüfkörperplättchen als Mass für die Säurebeständigkeit bestimmt.

### Bestimmung der Vickers-Härte

Die Härte ist ein Mass für die mechanische Beständigkeit von Werkstoffen. Da die eingesetzten Gläser Abrieb durch Kaukräfte ausgesetzt sind, ist eine Quantifizierung dieser Eigenschaft sinnvoll.

Zur Messung der Vickers-Härte wurden Prüfkörper mit einem Durchmesser von 20 mm und einer Dicke von 3 mm durch Zusammensintern des Glaspulvers mit einer mittleren Teilchengrösse von weniger als 50 µm hergestellt. Das Pulver wurde 1 Minute lang auf der jeweiligen Brenntemperatur für die Prüfkörperherstellung gehalten. Die Bestimmung der Vickers-Härte erfolgte nach ISO 14705 (2000) unter Verwendung des Mittelwertes aus 6 Messungen an polierten Oberflächen.

### Beispiel 5: Verwendung erfindungsgemäßen Glases als Glasurmaterial

Dieses Beispiel beschreibt die Verwendung des erfindungsgemässen Glases als Glasurmaterial bei Restaurationsteilen, die in einem nur teilweise verfestigtem Zustand geschliffen oder gefräst wurden und nachträglich einer weiteren thermischen Behandlung unterzogen werden, um die mechanischen Eigenschaften zu verbessern. Es wurde eine Restauration aus einer Lithiummetasilikat-Glaskeramik verwendet. Die thermische Behandlung führte überraschenderweise in einem Schritt nicht nur zu einer deutlichen Zunahme der Festigkeit, sondern gleichzeitig auch zur Glasur der Restauration. Das erfindungsgemäße Glas kann somit als Sofortglasur bezeichnet werden.

Glaspulver gemäss Beispiel 2 mit einer mittleren Korngrösse von maximal 50 µm wurde mit einem Universal Glasur- und Malfarbenliquid der Fa. Ivoclar Vivadent AG angemischt und auf eine mittels CAD/CAM-Technologie (Cerec^{®} 3 der Firma Sirona, Bensheim, Deutschland) hergestellte Modellkrone aus einer Lithiummetasilikat-Glaskeramik in einer durchschnittlichen Schichtdicke von ca. 150 µm aufgebracht. Anschliessend erfolgte die thermische Behandlung bei einer Temperatur von 860°C bei einer Aufheizrate von 60°C/min und einer Haltezeit von 10 min. Dabei wurde die Lithiummetasilikat-Glaskeramik in eine Lithiumdisilikat-Glaskeramik umgewandelt und gleichzeitig erfolgte der Glasurbrand mit dem erfindungsgemässen Glas.

Die Untersuchungen zur Festigkeit der eingesetzen und der nach der thermischen Behandlung erhaltenen Glaskeramik (Biaxiale Biegefestigkeit) brachten folgende Ergebnisse (Mittelwert aus 4 Messungen an nicht glasierten Prüfkörpern):
Lithiummetasilikat-Glaskeramik: 244 ± 27 MPa
Lithiumdisilikat-Glaskeramik: 537 ± 41 MPa

Weiterhin wurde der Glanz eines nicht glasierten und eines mit dem erfindungsgemässen Glas glasierten Prüfkörpers aus Lithiumdisilicat-Glaskeramik bestimmt (Mittelwert von 2 Prüfkörpern und je 4 Messungen):
Lithiumdisilikat-Glaskeramik (nicht glasiert): 5%
Lithiumdisilicat-Glaskeramik (glasiert): 60%

Die Bestimmung des Glanzes erfolgte mit dem Glanzmeßgerät novo-curve der Firma Rhopoint, Großbritannien, welches ein Glanzbestimmung selbst bei kleinen Proben gestattet. Die Messung erfolgte nach der Norm ISO 2813. Das Meßprinzip besteht darin, dass im Winkel von 60° ein Lichtstrahl auf die Oberfläche des Prüfkörpers gerichtet wird und die Intensität des reflektierten Lichtes im Gerät gemessen und mit einem Referenzwert verglichen wird.

### Beispiel 6:

Dieses Beispiel beschreibt die Verwendung des erfindungsgemäßen Glases als Glasurmaterial für eine Glimmer-Glaskeramik.

Ein Glasblock aus einem Glas, welches in der Lage ist Glimmer-kristalle auszuscheiden, wurde hierzu bei einer Temperatur von 750 °C für 1 Stunde getempert. Bei dieser Behandlung wurden im Glas durch Volumenkristallisation Chondroditkristalle ausgeschieden. Die Glaskeramik ließ sich in diesem Zustand mit Diamantwerkzeugen noch gut bearbeiten. Mittels CAD/CAM-Technologie (Cerec^{®} 3 der Firma Sirona, Bensheim, Deutschland) wurde aus ihr eine Modellkrone hergestellt.

Glaspulver gemäß Beispiel 1 mit einer mittleren Korngrösse von maximal 50 µm wurde mit einem Universal Glasur- und Malfarbenliquid der Firma Ivoclar Vivadent AG angemischt und auf die Modellkrone in einer durchschnittlichen Schichtdicke von ca. 150 µm aufgebracht. Anschliessend erfolgte die thermische Behandlung bei einer Temperatur von 950°C bei einer Aufheizrate von 60°C/min und einer Haltezeit von 20 min. Dabei wurde die Chondrodit-Glaskeramik in eine Glimmer-Glaskeramik umgewandelt und gleichzeitig erfolgte der Glasurbrand mit dem erfindungsgemäßen Glas.

Die Untersuchungen zur Festigkeit (Biaxiale Biegefestigkeit) brachten folgende Ergebnisse (Mittelwert aus 4 Messungen an nicht glasierten Prüfkörpern):
Chondrodit-Glaskeramik: 150 +/- 22 MPa
Glimmer-Glaskeramik: 310 +/- 42 MPa

## Patentansprüche

1. Glas für dentale Anwendungen, **dadurch gekennzeichnet, dass** es die folgenden Komponenten enthält
| Komponente | Gew.-% |
|---|---|
| SiO₂ | 60,0 - 65,0 |
| K₂O | 15,0 - 19,0 |
| Al₂O₃ | 6 , 0 - 10,5 |
| Li₂O | 1,8 - 2,2 |
| Me^{II}O | 2,5 - 8,5 |
| Weitere Oxide | 1,5 - 7,0 |
wobei
(a) Me^{II}O für mindestens ein zweiwertiges Oxid steht, das ausgewählt ist aus der Gruppe der Oxide von Ca, Mg, Sr und Zn, und
(b) weitere Oxide für mindestens ein Oxid steht, das aus der Gruppe der Oxide von B, Zr, Ce und Y ausgewählt ist.

2. Glas nach Anspruch 1, das im wesentlichen aus den Komponenten besteht.

3. Glas nach Anspruch 1 oder 2, das mindestens eine der Komponenten in der folgenden Menge enthält
| Komponent | Gew.-% |
|---|---|
| SiO₂ | 63.0 - 65.0 |
| K₂O | 17.5 - 19.0 |
| Al₂O₃ | 9.5 - 10.5 |
| Li₂O | 1.9 - 2.1 |
| Me^{II}O | 4.5 - 7.5 |
| Weitere Oxide | 2.5 - 6.0. |

4. Glas nach einem der Ansprüche 1 bis 3, das weniger als 2,0 Gew.-% an ZnO enthält.

5. Glas nach einem der Ansprüche 1 bis 4, das 0,5 bis 2,5 Gew.-% an ZrO₂ enthält.

6. Glas nach einem der Ansprüche 1 bis 5, das 15,5 bis 19,0, insbesondere 17,5 bis 19,0 Gew.-% an K₂O enthält.

7. Glas nach einem der Ansprüche 1 bis 6, das mindestens eine der Komponenten in der folgenden Menge enthält
| Komponente | Gew.-% |
|---|---|
| SiO₂ | 64.0 - 65.0 |
| K₂O | 18.0 - 18.5 |
| Al₂O₃ | 10.0 - 10.5 |
| Li₂O | 2.0 - 2.1 |
| MgO | 0.4 - 2.1, insbesondere 0.4 - 0.7 |
| CaO | 1.9 - 2.3, insbesondere 1.9 - 2.1 |
| ZrO₂ | 1.0 - 2.4, insbesondere 2.0 - 2.4 |
| CeO₂ | 0.4 - 0.8. |

8. Glas nach einem der Ansprüche 1 bis 7, das eine Brenntemperatur von 900 bis 950°C, insbesondere von 910 bis 930°C hat.

9. Glas nach einem der Ansprüche 1 bis 8, das einen linearen thermischen Ausdehnungskoeffizienten von 8,0 bis 9,5 10⁻⁶ K⁻¹ und insbesondere von 8,4 bis 9,1 · 10⁻⁶ K⁻¹ im Temperaturbereich von 100 bis 500°C hat.

10. Verwendung des Glases nach einem der Ansprüche 1 bis 9 als Glasurmaterial von dentalen Restaurationen.

11. Verwendung nach Anspruch 10, wobei die dentale Restauration eine Brücke, eine Krone, ein Inlay, ein Onlay, eine Verblendung, eine Kappe oder ein Teil von diesen ist.

12. Verwendung nach Anspruch 10 oder 11, wobei die dentale Restauration auf der Basis eines Glases, einer Keramik, eines Metalls oder einer Glaskeramik ist.

13. Verwendung nach Anspruch 12, wobei die Glaskeramik eine Lithiumsilicat-Glaskeramik, eine Glimmer-Glaskeramik oder eine Chondrodit-Glaskeramik ist.

14. Verwendung nach Anspruch 13, wobei die Lithiumsilicat-Glaskeramik eine Lithiummetasilicat- oder eine Lithiumdisilicat-Glaskeramik ist.

15. Verfahren zum Aufbringen einer Glasur auf eine dentale Restauration, bei dem
(a) das Glas nach einem der Ansprüche 1 bis 9 auf die dentale Restauration aufgebracht und
(b) die mit dem Glas versehende dentale Restauration einer thermischen Behandlung unterzogen wird, um das Glas in eine auf der Restauration fest haftende Glasur umzuwandeln.

16. Verfahren nach Anspruch 15, bei dem die thermische Behandlung bei einer Temperatur von wenigstens 860°C, insbesondere bei 860 bis 960°C erfolgt.

17. Verfahren nach Anspruch 15 oder 16, bei dem die dentale Restauration auf der Basis eines Glases, einer Keramik, eines Metalls oder einer Glaskeramik ist.

18. Verfahren nach Anspruch 17, bei dem die thermische Behandlung auch zur Bildung von Kristallen in dem Glas oder der Glaskeramik führt.

19. Verfahren nach Anspruch 18, bei dem die thermische Behandlung zur Bildung von Lithiumdisilicat-Kristallen oder Glimmer-Kristallen führt.

20. Verfahren nach Anspruch 18 oder 19, bei dem die Bildung von Kristallen eine Steigerung der Festigkeit, insbesondere der Biegefestigkeit, der dentalen Restauration bewirkt.

## Claims

1. Glass for dental applications comprising the following components
| Component | wt.-% |
|---|---|
| SiO₂ | 60.0 - 65.0 |
| K₂O | 15.0 - 19.0 |
| Al₂O₃ | 6.0 - 10.5 |
| Li₂O | 1.8 - 2.2 |
| Me^{II}O | 2.5 - 8.5 |
| further oxides | 1.5 - 7.0 |
wherein
(a) Me^{II}O represents at least one divalent oxide which is selected from the group of the oxides of Ca, Mg, Sr and Zn, and
(b) further oxides represents at least one oxide which is selected from the group of the oxides of B, Zr, Ce and Y.

2. Glass according to claim 1, which essentially consists of the components.

3. Glass according to claim 1 or 2, which comprises at least one of the components in the following quantity
| Component | wt.-% |
|---|---|
| SiO₂ | 63.0 - 65.0 |
| K₂O | 17.5 - 19.0 |
| Al₂O₃ | 9.5 - 10.5 |
| Li₂O | 1.9 - 2.1 |
| Me^{II}O | 4.5 - 7.5 |
| Further oxides | 2.5 - 6.0. |

4. Glass according to any one of claims 1 to 3, which comprises less than 2.0 wt.-% ZnO.

5. Glass according to any one of claims 1 to 4, which comprises 0.5 to 2.5 wt.-% ZrO₂.

6. Glass according to any one of claims 1 to 5, which comprises 15.5 to 19.0, in particular 17.5 to 19.0 wt.-% K₂O.

7. Glass according to any one of claims 1 to 6, which comprises at least one of the components in the following amount
| Component | wt.-% |
|---|---|
| SiO₂ | 64.0 - 65.0 |
| K₂O | 18.0 - 18.5 |
| Al₂O₃ | 10.0 - 10.5 |
| Li₂O | 2.0 - 2.1 |
| MgO | 0.4 - 2.1, in particular 0.4 - 0.7 |
| CaO | 1.9 - 2.3, in particular 1.9 - 2.1 |
| ZrO₂ | 1.0 - 2.4, in particular 2.0 - 2.4 |
| CeO₂ | 0.4 - 0.8. |

8. Glass according to any one of claims 1 to 7, which has a firing temperature of 900 to 950°C, in particular of 910 to 930°C.

9. Glass according to any one of claims 1 to 8, which has a linear coefficient of thermal expansion of 8.0 to 9.5 - 10⁻⁶ K⁻¹ and in particular of 8.4 to 9.1 - 10⁻⁶ K⁻¹ in the temperature range of 100 to 500°C.

10. Use of the glass according to any one of claims 1 to 9 as glazing material for dental restorations.

11. Use according to claim 10, wherein the dental restoration is a bridge, a crown, an inlay, an onlay, a veneer, a cap, or a part thereof.

12. Use according to claim 10 or 11, wherein the dental restoration is based on a glass, a ceramic, a metal or a glass-ceramic.

13. Use according to claim 12, wherein the glass-ceramic is a lithium silicate glass-ceramic, a mica glass-ceramic or a chondrodite glass-ceramic.

14. Use according to claim 13, wherein the lithium silicate glass-ceramic is a lithium metasilicate or a lithium disilicate glass-ceramic.

15. Process for applying a glaze on a dental restoration, in which
(a) the glass according to any one of claims 1 to 9 is applied onto the dental restoration and
(b) the dental restoration provided with the glass is subjected to a thermal treatment in order to transform the glass into a glaze which adheres firmly to the restoration.

16. Process according to claim 15, in which the thermal treatment takes place at a temperature of at least 860°C, in particular at 860 to 960°C.

17. Process according to claim 15 or 16, in which the dental restoration is based on a glass, a ceramic, a metal or a glass-ceramic.

18. Process according to claim 17, in which the thermal treatment also leads to the formation of crystals in the glass or the glass-ceramic.

19. Process according to claim 18, in which the thermal treatment leads to the formation of lithium disilicate crystals or mica crystals.

20. Process according to claim 18 or 19, in which the formation of crystals brings about an increase in the strength, in particular the bending strength, of the dental restoration.

## Revendications

1. Verre pour utilisations dentaires, **caractérisé en ce qu'**il contient les composants suivants :
| Composant | % en poids |
|---|---|
| SiO₂ | 60,0 - 65,0 |
| K₂O | 15,0 - 19,0 |
| Al₂O₃ | 6,0 - 10,5 |
| Li₂O | 1,8 - 2,2 |
| Me^{II}O | 2,5 - 8,5 |
| Autres oxydes | 1,5 - 7,0 |
parmi lesquels :
a) Me^{II}O représente au moins un oxyde de métal divalent, choisi parmi les oxydes de calcium, de magnésium, de strontium et de zinc,
b) et les "autres oxydes", qui sont au nombre d'au moins un, sont choisis parmi les oxydes de bore, de zirconium, de cérium et d'yttrium.

2. Verre conforme à la revendication 1, constitué pour l'essentiel des composants indiqués.

3. Verre conforme à la revendication 1 ou 2, dans lequel au moins l'un de ses composants est contenu en la proportion respective indiquée ci-dessous :
| Composant | % en poids |
|---|---|
| SiO₂ | 63,0 - 65,0 |
| K₂O | 17,5 - 19,0 |
| Al₂O₃ | 9,5 - 10,5 |
| Li₂O | 1,9 - 2,1 |
| Me^{II}O | 4,5 - 7,5 |
| Autres oxydes | 2,5 - 6,0. |

4. Verre conforme à l'une des revendications 1 à 3, qui contient moins de 2,0 % en poids d'oxyde de zinc ZnO.

5. Verre conforme à l'une des revendications 1 à 4, qui contient de 0,5 à 2,5 % en poids d'oxyde de zirconium ZrO₂.

6. Verre conforme à l'une des revendications 1 à 5, qui contient de 15,5 à 19,0 % et en particulier de 17,5 à 19,0 % en poids d'oxyde de potassium K₂O.

7. Verre conforme à l'une des revendications 1 à 6, dans lequel au moins l'un de ses composants est contenu en la proportion respective indiquée ci-dessous :
| Composant | % en poids |
|---|---|
| SiO₂ | 64,0 - 65,0 |
| K₂O | 18,0 - 18,5 |
| Al₂O₃ | 10,0 - 10,5 |
| Li₂O | 2,0 - 2,1 |
| MgO | 0,4 - 2,1 en particulier 0,4 - 0,7 |
| CaO | 1,9 - 2,3 en particulier 1,9 - 2,1 |
| ZrO₂ | 1,0 - 2,4 en particulier 2,0 - 2,4 |
| CeO₂ | 0,4 - 0,8. |

8. Verre conforme à l'une des revendications 1 à 7, dont la température de cuisson vaut de 900 à 950 °C, en particulier de 910 à 930 °C.

9. Verre conforme à l'une des revendications 1 à 8, dont le coefficient linéaire de dilatation thermique vaut de 8,0.10⁻⁶ à 9,5.10⁻⁶ K⁻¹, en particulier de 8,4.10⁻⁶ à 9,1.10⁻⁶ K⁻¹, dans l'intervalle de température allant de 100 à 500 °C.

10. Utilisation d'un verre conforme à l'une des revendications 1 à 9 comme matériau d'émail de restauration dentaire.

11. Utilisation conforme à la revendication 10, pour la quelle la restauration dentaire est un bridge, une couronne, une prothèse intrinsèque, une prothèse extrinsèque, une facette ou une coiffe, ou une partie d'une telle restauration.

12. Utilisation conforme à la revendication 10 ou 11, pour laquelle la restauration dentaire est à base de verre, de céramique, de métal ou de vitrocéramique.

13. Utilisation conforme à la revendication 12, pour laquelle la vitrocéramique est une vitrocéramique de silicate de lithium, une vitrocéramique de mica ou une vitrocéramique de chondrodite.

14. Utilisation conforme à la revendication 13, pour laquelle la vitrocéramique de silicate de lithium est une vitrocéramique de métasilicate de lithium ou une vitrocéramique de disilicate de lithium.

15. Procédé de dépôt d'un émail sur une restauration dentaire, dans lequel
a) on dépose sur la restauration dentaire un verre conforme à l'une des revendications 1 à 9,
b) et l'on soumet la restauration dentaire munie de ce verre à un traitement thermique, pour transformer le verre en un émail qui adhère solidement à la restauration.

16. Procédé conforme à la revendication 15, dans lequel on opère le traitement thermique à une température d'au moins 860 °C, en particulier de 860 à 960 °C.

17. Procédé conforme à la revendication 15 ou 16, dans lequel la restauration dentaire est à base de verre, de céramique, de métal ou de vitrocéramique.

18. Procédé conforme à la revendication 17, dans lequel le traitement thermique entraîne également la formation de cristaux dans le verre ou la vitrocéramique.

19. Procédé conforme à la revendication 18, dans lequel le traitement thermique entraîne également la formation de cristaux de disilicate de lithium ou de cristaux de mica.

20. Procédé conforme à la revendication 18 ou 19, dans lequel la formation de cristaux a pour conséquence une augmentation de la résistance mécanique de la restauration dentaire, en particulier de sa résistance à la flexion.
